# EUROPÄISCHE PATENTSCHRIFT

(11) **EP 0 522 312 B1**
(45) Veröffentlichungstag und Bekanntmachung des Hinweises auf die Patenterteilung: **08.03.1995**
(21) Anmeldenummer: 92110016.0
(22) Anmeldetag: 13.06.1992
(51) Int. Cl.: C07C 43/307, C25B 3/02

(54) **o-Phthaldialdehydtetraalkylacetale, ihre Herstellung und ihre Verwendung als Depotverbindung**
o-Phthaldialdehyde-tetraalkyl acetals, their preparation and their use as depository compounds
Tétraalcoylacétals de o-phthaldialdéhyde, leur préparation et leur utilisation comme agent de dépôt

(30) Priorität: 05.07.1991 DE 4122314
(43) Veröffentlichungstag der Anmeldung: 13.01.1993
(73) Patentinhaber: BASF Aktiengesellschaft, 67063 Ludwigshafen (DE)
(72) Erfinder: Hermeling, Dieter, Dr., W-6710 Frankenthal (DE)

(56) Entgegenhaltungen:
- EP-A- 0 012 240
- EP-A- 0 164 705
- DE-A- 3 108 790
- ELECTROCHIMICA ACTA, Bd. 29, Nr. 12, Dezember 1984, Oxford, GB, Seiten 1639 - 1641; F. BARBA et al: "Electrochemical Methoxylations of Side-Chain Methoxylated p-Xylene Derivatives"
- CHEMICAL ABSTRACTS, vol. 103, no. 10, September 1985, Columbus, Ohio, US; abstract no. 78306u, "Terephthalaldehyde Electrochemical Production", Seite 470; & JP-A-60030183

## Beschreibung

Die Erfindung betrifft neue o-Phthaldialdehydtetraalkylacetale, ein Verfahren zu deren Herstellung durch elektrochemische Oxidation sowie ihre Verwendung als Depotverbindungen für die entsprechenden Aldehyde.

Acetale sind Verbindungen, die nicht nur in der Schutzgruppenchemie von besonderem Interesse sind, weil sie die reaktiven Aldehyde vor einer ungewollten Umsetzung schützen, sie dienen auch als thermisch und oxidativ stabile Depotverbindungen für Aldehyde, die bei Bedarf unter milden Bedingungen quantitativ aus den Acetalen freigesetzt werden können.

Von einem besonderen Interesse sind Phthaldialdehyde, die z. B. nach DE 3 421 976 A1 als Zwischenprodukte für die Herstellung von Farbstoffen, optischen Aufhellern oder speziellen Polymeren dienen.

o-Phthaldialdehydtetraalkylacetale können z.B. nach der DE 3 108 790 A1 durch Umsetzung von α, α, α', α'-Tetrahalogenxylolen mit Alkalialkoholaten hergestellt werden. Dieses Verfahren hat aber den Nachteil, daß die Tetrahalogenxylole toxikologisch bedenklich und nur in unbefriedigenden Selektivitäten zugänglich sind.

Nach DE 3 421 976 A1 lassen sich Phthaldialdehydtetraalkylacetale elektrochemisch durch Oxidation der entsprechenden Bis-(alkoxymethyl)benzole herstellen. Da diese Einsatzstoffe aber aus Bis-(halogenmethyl)benzolen synthetisiert werden, ergeben sich auch in diesem Fall die oben genannten Probleme.

Der Erfindung lag daher die Aufgabe zugrunde, o-Phthaldialdehydtetraalkylacetale in einem einfachen Verfahren zur Verfügung zu stellen, ohne daß halogenhaltige Ausgangsprodukte eingesetzt werden müssen.

Es wurde nun gefunden, daß substituierte o-Xylole der allgemeinen Formel II 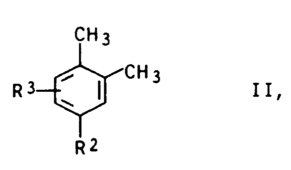
in der R² einen C₃-C₂₀-Alkyl-, einen C₃-C₂₀-Cycloalkyl- oder ein C₄-C₂₀-Alkyl-Cycloalkylrest und R³ Wasserstoff oder R² bedeuten, in Gegenwart eines Alkohols R¹OH, wobei R¹ für einen C₁-C₆-Alkyl-Rest steht, zu o-Phthaldialdehydtetraalkylacetalen der allgemeinen Formel I 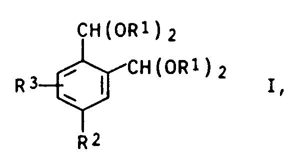
in der R¹, R² und R³ die o. g. Bedeutung haben, elektrochemisch oxidiert werden können, so daß nun ein umweltfreundlicher Zugang zu o-Phthaldialdehydtetraalkylacetalen ohne halogenhaltige Vorstufen möglich ist.

Gegenstand der vorliegenden Erfindung sind somit o-Phthaldialdehydtetraalkylacetale der allgemeinen, oben angegebenen Formel I.

Die Elektrooxidation substituierter und unsubstituierter Xylole führt nach EP 12 240 A2 zu Methylbenzaldehyddialkylacetalen. Nach J. Chem. Research 1986, 228 - 229 werden die Acetale, wie am Beispiel der Elektrolyse von o-Xylol beschrieben, im anschließenden Oxidationsschritt zum entsprechenden Orthoester weiteroxidiert, während die zweite Methylgruppe unverändert bleibt.

Unter diesem Gesichtspunkt war es überraschend, daß die substituierten Xylole der Formel II in guten Ausbeuten zu den o-Phthaldialdehydtetraalkylacetalen der Formel I elektrochemisch oxidiert werden können.

Die vorliegende Erfindung betrifft demnach auch ein Verfahren, bei dem man substituierte Xylole der allgemeinen, oben angegebenen Formel II elektrochemisch zu o-Phthaldialdehydtetraalakylacetalen oxidiert.

Als Alkohole der Formel R¹OH werden solche mit 1 - 6 C-Atomen, bevorzugt 1 - 4 C-Atomen eingesetzt. Besonders bevorzugt sind Methanol und Ethanol.

Für die Substituenten R² und R³ ist es bevorzugt, daß die α-C-Atome keinen Wasserstoff tragen, da das Vorhandensein von Wasserstoff am α-C-Atom zu Nebenprodukten bei der elektrochemischen Oxidation führt und somit die Ausbeuten sinken.

Als Substituenten R² kommen Alkylreste mit 3 - 20, vorzugsweise 4 - 12 C-Atomen, wie n-Butyl, iso-Butyl, tert.-Butyl, n-Pentyl, tert.-Amyl, 1-Methylbut-1-yl, 2,2-Dimethylprop-1-yl, n-Hexyl, 1-Methylpent-1-yl, 1,1-Dimethylbut-1-yl, 1,1,2-Trimethylprop-1-yl, 1-Ethyl-1-methylprop-1-yl, 2,2-Diethyleth-1-yl, n-Heptyl, 1-Methylhex-1-yl, 1,1-Dimethylpent-1-yl, 1-Ethyl-1-methylbut-1-yl, 1,1-Diethylprop-1-yl, 1-Methyl-1-propylprop-1-yl, 1-Ethyl-1-propyleth-1-yl, 1-Methyl-1-butyleth-1-yl, 1,1,2,2-Tetramethylprop-1-yl, n-Octyl, 1,1-Dimethylhex-1-yl, 1-Ethyl-1-methylpent-1-yl, 1,1-Diethylbut-1-yl, 1-Methyl-1-propylbut-1-yl, 1-Ethyl-1-propylprop-1-yl, 1,1,3,3-Tetramethylbut-1-yl, 1,1-Dimethylhept-1-yl, 1,1-Dimethyloct-1-yl, 1,1-Dimethylnon-1-yl, 1,1-Dimethyldec-1-yl, besonders bevorzugt tert.-Butyl, tert.-Amyl, 1,1-Dimethylbut-1-yl, 1,1,2-Trimethylprop-1-yl, 1,1-Dimethylpent-1-yl, 1,1,2,2-Tetramethylprop-1-yl,
Cycloalkylreste mit 3 - 20, vorzugsweise 5 - 12 C-Atomen, wie Cyclopentyl, Cyclohexyl, Cycloheptyl, Cyclooctyl, Cyclononyl, Cyclodecyl, Cycloundecyl, Cyclododecyl, besonders bevorzugt Cyclopentyl, Cyclohexyl, Cycloheptyl, oder
Alkyl-Cycloalkylreste mit 4 - 20, vorzugsweise 6 - 12 C-Atomen, wie 1-Methylcyclopent-1-yl, 1-Methylcyclohex-1-yl, 1-Ethylcyclohex-1-yl, 1,4-Dimethylcyclohex-1-yl, 1,3-Dimethylcyclohex-1-yl, 1,3,5-Trimethylcyclohex-1-yl, 1-Isopropylcyclohex-1-yl, 1-tert.-Butylcyclohex-1-yl, besonders bevorzugt 1-Methylcyclopent-1-yl, 1-Methyl-cyclohex-1-yl, 1,3,5-Trimethylcyclohex-1-yl in Frage.

Der Substituent R³ steht für Wasserstoff oder R².

Bevorzugte erfindungsgemäße Verbindungen sind:
4-tert.-Butyl-1,2-bis-(dimethoxymethyl)benzol
4-tert.-Amyl-1,2-bis-(dimethoxymethyl)benzol
4-(1,1-Dimethylbutyl)-1,2-bis-(dimethoxymethyl)benzol
4-(1,1,2-Trimethylpropyl)-1,2-bis-(dimethoxymethyl)benzol
4-(1,1-Dimethylpentyl)-1,2-bis-(dimethoxymethyl)benzol
4-(1,1,2,2-Tetramethylpropyl)-1,2-bis-(dimethoxymethyl)benzol
4-(1,1-Dimethylhexyl)1,2-bis-(dimethoxymethyl)benzol
4-(1,1,3,3-Tetramethylbutyl)-1,2-bis-(dimethoxymethyl)benzol
4-(1,1-Dimethylheptyl)-1,2-bis-(dimethoxymethyl)benzol
4-(1,1-Dimethyloctyl)-1,2-bis-(dimethoxymethyl)benzol
4-(1-Methylcyclopentyl)-1,2-bis(dimethoxymethyl)benzol
4-(1-Methylcyclohexyl)-1,2-bis-(dimethoxymethyl)benzol
4-(1,4-Dimethylcyclohexyl)-1,2-bis-(dimethoxymethyl)benzol
4-(1,3,5-Trimethylcyclohexyl)-1,2-bis-(dimethoxymethyl)benzol
4-tert.-Butyl-1,2-bis-(diethoxymethyl)benzol
4-tert.-Amyl-1,2-bis-(diethoxymethyl)benzol
4-(1,1-Dimethylbutyl)-1,2-bis-(diethoxymethyl)benzol
4-(1,1,2-Trimethylpropyl)-1,2-bis(diethoxymethyl)benzol
4-(1,1-Dimethylpentyl)-1,2-bis-(diethoxymethyl)benzol
4-(1,1,2,2,-Tetramethylpropyl)-1,2-bis-(diethoxymethyl)benzol
4-(1,1-Dimethylhexyl)-1,2-bis-(diethoxymethyl)benzol
4-(1,1,3,3-Tetramethylbutyl)-1,2-bis-(diethoxymethyl)benzol
4-(1,1-Dimethylheptyl)-1,2-bis-(diethoxymethyl)benzol
4-(1,1-Dimethyloctyl)-1,2-bis-(diethoxymethyl)benzol
4-(1-Methylcyclopentyl)-1,2-bis-(diethoxymethyl)benzol
4-(1-Methylcyclohexyl)-1,2-bis-(diethoxymethyl)benzol
4-(1,4-Dimethylcyclohexyl)-1,2-bis(diethoxymethyl)benzol
4-(1,3,5-Trimethylcyclohexyl)-1,2-bis-(diethoxymethyl)benzol
Die Verbindungen der allg. Formel I sind neu und können in einem elektrochemischen Verfahren in technisch üblichen Elektrolysezellen hergestellt werden.

Bevorzugt werden ungeteilte Durchflußzellen verwendet. Als Anoden kommen z. B. Edelmetallelektroden wie Platin oder Oxidelektroden wie Ti/RuOₓ, RuO₂ oder Cr₂O₃ in Frage. Bevorzugtes Anodenmaterial ist Graphit. Als Kathoden kommen z. B. Stahl, Eisen, Nickel, Kupfer, Zink und Kohle sowie Edelmetalle wie Platin in Betracht. Bevorzugtes Kathodenmaterial ist Graphit. Der Elektrolyt setzt sich aus der Ausgangsverbindung der Formel II, dem Alkohol R¹OH und einem Hilfselektrolyten zusammen. Als Hilfselektrolyte kommen Neutralsalze, Säuren und Basen in Betracht. Beispiele für Neutralsalze sind Fluoride wie KF, Sulfonate wie NaSO₃Ph, Sulfate wie (CH₃)₄NSO₄CH₃, Tetrafluoroborate wie NaBF₄, Phosphate und Phosphonate.

Beispiele für Säuren sind Schwefelsäure, Alkyl- und Arylsulfonsäuren wie Methyl- oder Benzolsulfonsäure. Als Basen dienen z.B. Alkoholate wie NaOCH₃ oder Hydroxide wie KOH.

Der Elektrolyt hat beispielweise folgende Zusammensetzung:
1 bis 49, vorzugsweise 5 - 30 Gew.-% Verbindung der Formel II
50 - 98,9 vorzugsweise 70 - 95 Gew.-% R¹OH
0,1 - 5, vorzugsweise 0,2 - 3 Gew.-% Hilfselektrolyt.

Die Stromdichte kann bei dem erfindungsgemäßen Verfahren in weiten Grenzen, z. B. von 0,1 - 25 A/dm², bevorzugt von 1 - 10 A/dm² gewählt werden. Auch die Temperaturen können in weiten Grenzen variiert werden. So können die Oxidationen bei 0 - 100°C, bevorzugt bei 20 - 80°C durchgeführt werden. Die Elektrolysetemperatur ist u. a. vom Alkohol R¹OH abhängig. Es wird in jedem Fall bei Temperaturen unterhalb des Siedepunktes des Alkohols R¹OH gearbeitet. Die Elektrolysen werden bevorzugt unter Normaldruck durchgeführt, es kann aber auch unter einem Überdruck bis zu 10 bar elektrolysiert werden. Durch die damit verbundene Siedepunktserhöhung kann z. B auch in Methanol bei Temperaturen oberhalb von 60°C elektrolysiert werden.

Die Ausgangsverbindungen der Formel II lassen sich, soweit sie nicht käuflich erhältlich sind, ohne weiteres in an sich bekannter Weise durch Alkylierung von Xylol herstellen.

Die Ausgangsverbindungen der Formel II lassen sich weitestgehend umsetzen. Nicht umgesetztes Edukt sowie bei der Elektrolyse auftretende Zwischenprodukte können in die Elektrolyse zurückgeführt werden. Die Elektrolyse kann sowohl kontinuierlich als auch diskontinuierlich durchgeführt werden. Die Aufarbeitung der Elektrolyseausträge erfolgt nach konventionellen Methoden, vorzugsweise wird destillativ aufgearbeitet.

Die erfindungsgemäßen o-Phthaldialdehydtetraalkylacetale eignen sich als stabile Depotverbindungen für die entsprechenden o-Phthaldialdehyde, da sich die Acetale durch Hydrolyse in an sich bekannter Weise in die Aldehyde überführen lassen (siehe z.B. Houben-Weyl, Meth. d. org. Chemie, 4. Aufl., Bd. 7/1, Sauerstoffverbindungen II, S. 423-428; G.-Thieme-Verlag, Stuttgart, 1954).

### Beispiele

### Beispiel 1

### Elektrosynthese von 4-tert.-Butyl-1,2-bis-(dimethoxymethyl)benzol

- Apparatur:: ungeteilte Zelle mit 11 bipolaren Elektroden
- Anoden:: Graphit
- Elektrolyt:: 450 g (2,778 mol) 4-tert.-Butyl-1,2-dimethylbenzol, 30 g Natriumbenzolsulfonat und 2520 g Methanol
- Kathoden:: Graphit
- Stromdichte:: 3,4 A/dm²
- Elektrolysetemperatur:: 46°C

Elektrolyse mit 17 F/mol 4-tert.-Butyl-1,2-dimethylbenzol.

Der Elektrolyt wird während der Elektrolyse mit 200 l/h durch die Zelle gepumpt.

### Aufarbeitung:

Nach Beendigung der Elektrolyse wird das Methanol bei Normaldruck bis zu einer Sumpftemperatur von 120°C abdestilliert, das Leitsalz abfiltriert und das Filtrat im Vakuum reindestilliert. Man erhält 467 g (1,656 Mol) 4-tert.-Butyl-1,2-bis-(dimethoxymethyl)benzol.
Ausbeute: 57 %
¹³C-NMR (CDCl₃)
δ (ppm) = 31,4 (q, 3C), 34,7 (s), 53,3 (q, 2C), 53,4 (q, 2C), 101,5 (d), 101,8 (d), 123,7 (d) 125,0 (d), 126,8 (d), 133,3 (s), 135,6 (s) 151,2 (s).

### Beispiel 2

### Elektrosynthese von 4-tert.-Amyl-1,2-bis-(dimethoxymethyl)benzol

4-tert.-Amyl-1,2-dimethylbenzol wird in der in Beispiel 1 beschriebenen Elektrolysezelle unter den dort angegebenen Bedingungen oxidiert.
Elektrolyt.:
1135 g (6,449 mol) 4-tert.-Amyl-1,2-dimethylbenzol, 30 g Natriumbenzolsulfonat und 2520 g Methanol.
Elektrolysetemperatur: 31 - 33°C
Elektrolyse mit 16 F/mol 4-tert.-Amyl-1,2-dimethylbenzol.

Die Aufarbeitung des Elektrolyten erfolgt wie in Beipsiel 1 beschrieben. Nach Reindestillation im Vakuum werden 1002 g (3,385 mol) 4-tert.-Amyl-1,2-bis-(dimethoxymethyl)benzol erhalten.
Ausbeute: 53 %
¹³C-NMR (CDCl₃)
δ (ppm) = 9,1 (q), 28,5 (q, 2C), 36,9 (t), 38,0 (s), 53,4 (q, 4C), 101,6 (d), 101,8 (d), 124,5 (d), 125,8 (d), 126,7 (d), 133,3 (s), 135,5 (s), 149,6 (s).

## Patentansprüche

1. o-Phthaldialdehydtetraalkylacetale der allgemeinen Formel I 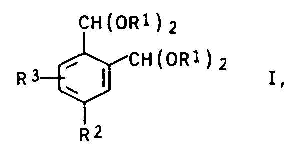 in der R¹ einen C₁-C₆-Alkyl-, R² einen C₃-C₂₀-Alkyl-, einen C₃-C₂₀-Cycloalkyl- oder einen C₄-C₂₀-Alkyl-Cycloalkyl-Rest und R³ Wasserstoff oder R² bedeuten.

2. o-Phthaldialdehydtetraalkylacetale nach Anspruch 1, dadurch gekennzeichnet, daß R² und R³ C₄-C₂₀-Alkyl- oder C₄-C₂₀-Alkyl-Cycloalkyl-Reste ohne einen Wasserstoff am α-C-Atom sind.

3. o-Phthaldialdehydtetraalkylacetale nach Anspruch 2, dadurch gekennzeichnet, daß R² der tert.-Butyl- oder der tert.-Amyl-Rest und R¹ der Methyl- oder Ethyl-Rest und R³ Wasserstoff ist.

4. Verfahren zur Herstellung von o-Phthaldialdehydtetraalkylacetalen der allgemeinen Formel I 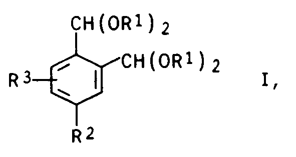 in der R¹, R² und R³ die in Anspruch 1 genannte Bedeutung haben, dadurch gekennzeichnet, daß man substituierte Xylole der allgemeinen Formel II 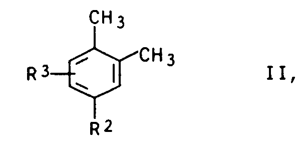 in der R² und R³ die in Anspruch 1 genannte Bedeutung haben, in Gegenwart eines Alkohols R¹OH, wobei R¹ für ein C₁-C₆-Alkyl steht, elektrochemisch oxidiert.

5. Verfahren nach Anspruch 4, dadurch gekennzeichnet, daß man die Elektrolysen in ungeteilten Zellen durchführt.

6. Verfahren nach Anspruch 4, dadurch gekennzeichnet, daß man die Elektrolysen an Graphitanoden durchführt.

7. Verfahren nach Anspruch 4, dadurch gekennzeichnet, daß man die Elektrolysen bei Temperaturen von 0 - 100°C bei Normaldruck oder unter Überdruck durchführt.

8. Verfahren nach Anspruch 4, dadurch gekennzeichnet, daß man die Elektrolysen bei Stromdichten von 0,1 - 25 A/dm² durchführt.

9. Verwendung der o-Phthaldialdehydtetraalkylacetale nach Anspruch 1 als stabile Depotverbindungen für die o-Phthaldialdehyde.

## Claims

1. A phthalaldehyde tetraalkyl acetal of the formula I 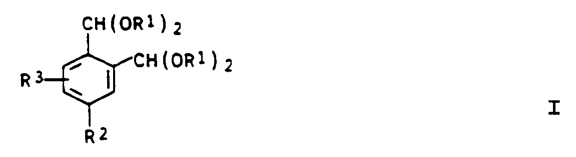 where R¹ is C₁-C₆-alkyl, R² is C₃-C₂₀-alkyl, C₃-C₂₀-cycloalkyl or C₄-C₂₀-alkyl-cycloalkyl, and R³ is hydrogen or R².

2. A phthalaldehyde tetraalkyl acetal as claimed in claim 1, where R² and R³ are C₄-C₂₀-alkyl or C₄-C₂₀-alkyl-cycloalkyl without a hydrogen on the α-C atom.

3. A phthalaldehyde tetraalkyl acetal as claimed in claim 2, where R² is tert-butyl or tert-amyl, R¹ is methyl or ethyl and R³ is hydrogen.

4. A process for preparing phthalaldehyde tetraalkyl acetals of the formula I 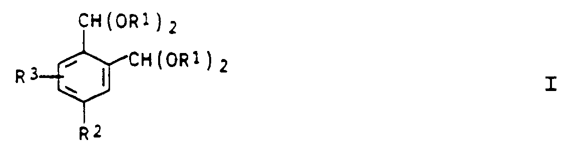 where R¹, R² and R³ have the meanings specified in claim 1, which comprises electrochemical oxidation of substituted xylenes of the formula II 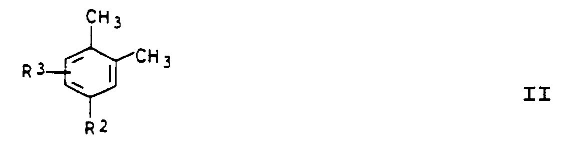 where R² and R³ have the meanings specified in claim 1, in the presence of an alcohol R¹OH where R¹ is C₁-C₆-alkyl.

5. A process as claimed in claim 4, wherein the electrolyses are carried out in undivided cells.

6. A process as claimed in claim 4, wherein the electrolyses are carried out at graphite anodes.

7. A process as claimed in claim 4, wherein the electrolyses are carried out at 0-100°C under atmospheric pressure or superatmospheric pressure.

8. A process as claimed in claim 4, wherein the electrolyses are carried out with current densities of 0.1-25 A/dm².

9. A method of using the phthalaldehyde tetraalkyl acetals as claimed in claim 1 as stable compounds for storing phthalaldehydes.

## Revendications

1. Tétraalkylacétals de dialdéhyde o-phtalique de formule générale I 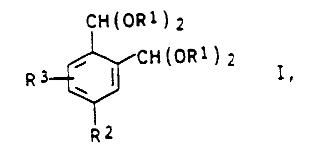 dans laquelle R¹ représente un reste alkyle en C₁-C₆, R² représente un reste alkyle en C₃-C₂₀, cycloalkyle en C₃-C₂₀ ou alkylcycloalkyle en C₄-C₂₀ et R³ représente un atome d'hydrogène ou R².

2. Tétraalkylacétals de dialdéhyde o-phtalique selon la revendication 1, caractérisés en ce que R² et R³ sont des restes alkyle en C₄-C₂₀ ou alkylcycloalkyle en C₄-C₂₀, sans atome d'hydrogène sur l'atome de carbone en α.

3. Tétraalkylacétals de dialdéhyde o-phtalique selon la revendication 2, caractérisés en ce que R² est le reste tert.-butyle ou tert.-amyle, R¹ est le reste méthyle ou éthyle et R³ est un atome d'hydrogène.

4. Procédé de préparation de tétraalkylacétals de dialdéhyde o-phtalique de formule générale I 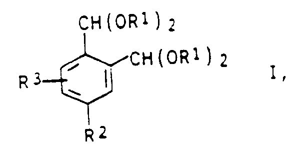 dans laquelle R¹, R² et R³ ont les significations indiquées dans la revendication 1, caractérisé en ce que l'on oxyde par voie électrochimique des xylènes substitués de formule générale II 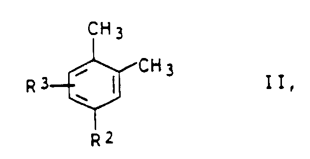 dans laquelle R² et R³ ont les significations indiquées dans la revendication 1, en présence d'un alcool R¹OH, R¹ étant mis pour un reste alkyle en C₁-C₆.

5. Procédé selon la revendication 4, caractérisé en ce qu'on effectue les électrolyses dans des cellules non séparées.

6. Procédé selon la revendication 4, caractérisé en ce qu'on effectue les électrolyses sur des anodes en graphite.

7. Procédé selon la revendication 4, caractérisé en ce qu'on effectue les électrolyses à des températures de 0 - 100°C, sous la pression normale ou sous surpression.

8. Procédé selon la revendication 4, caractérisé en ce qu'on effectue les electrolyses avec des densités de courant de 0,1 - 25 A/dm².

9. Utilisation des tétraalkylacétals de dialdéhyde o-phtalique selon la revendication 1 comme composés stables de mise en dépôt pour les dialdéhydes o-phtaliques.
